Europäisches Patentamt

European Patent Office

Office européen des brevets

(19)

(11) Veröffentlichungsnummer: **0 079 482**

**A1**

(12) # EUROPÄISCHE PATENTANMELDUNG

(21) Anmeldenummer: **82109748.2**

(22) Anmeldetag: **22.10.82**

(51) Int. Cl.³: **G 01 M 5/00**

(30) Priorität: **26.10.81 DE 3142351**

(43) Veröffentlichungstag der Anmeldung:
**25.05.83 Patentblatt 83/21**

(84) Benannte Vertragsstaaten:
**AT CH DE FR LI SE**

(71) Anmelder: **Hoch, Alois**
**Schwärzenbachstrasse 7**
**D-7450 Hechingen-Boll(DE)**

(72) Erfinder: **Hoch, Alois**
**Schwärzenbachstrasse 7**
**D-7450 Hechingen-Boll(DE)**

(72) Erfinder: **Ott, Fritz**
**Dorfstrasse 2**
**D-7450 Hechingen-Boll(DE)**

(74) Vertreter: **Commentz, Gerd, Dipl.-Ing.**
**Klippeneckstrasse 4**
**D-7000 Stuttgart 1(DE)**

(54) **Gerät zum Messen der Elastizität von Langlaufskiern.**

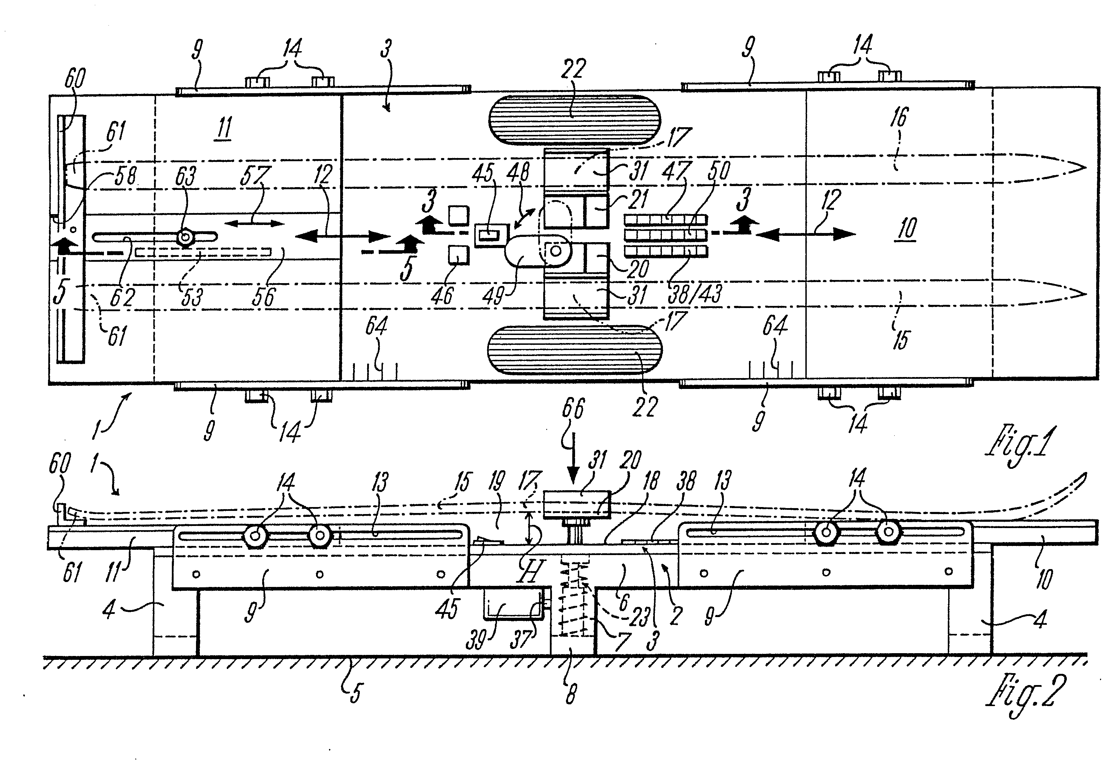

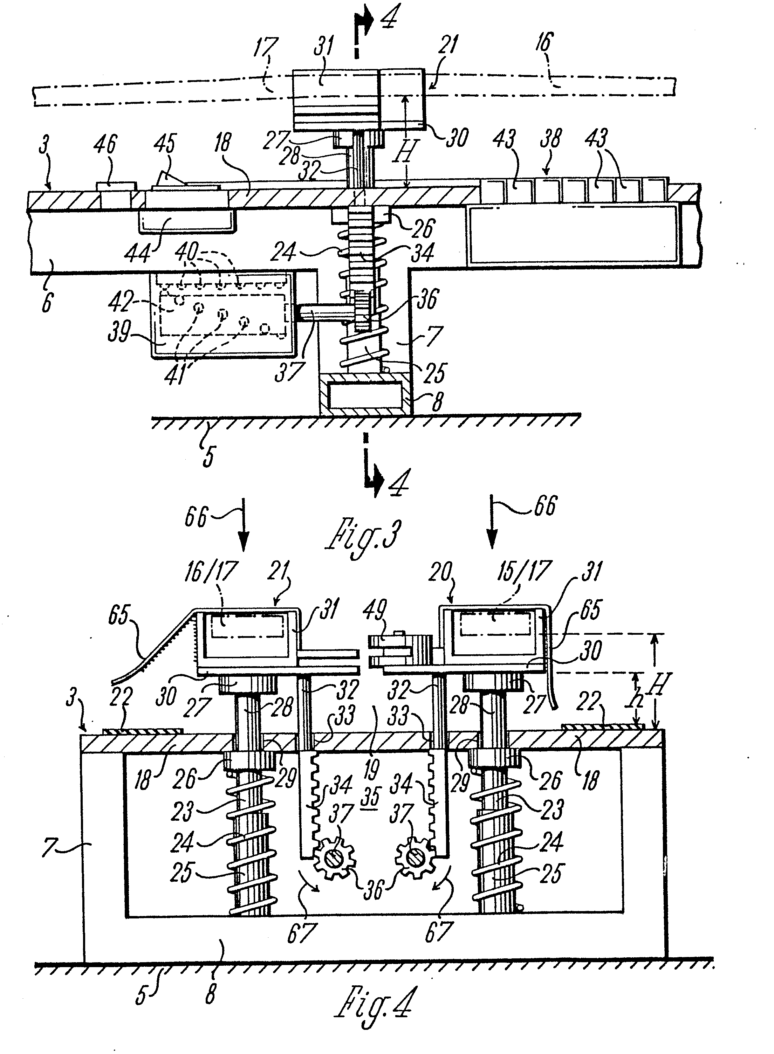

(57) Die Fig. 2 zeigt ein Gerät (1) zum Messen der Elastizität von Langlaufski (15), bei der mittels einer Abtasteinrichtung (20) das Absenken des durch das Eigengewicht einer Person belasteten Skimittelteiles auf eine Anzeigeeinrichtung (38) übertragen wird und somit festgestellt werden kann, ob bezogen auf das Eigengewicht der betreffenden Person der Ski (15) die richtige Elastizität aufweist oder aber diese zu groß oder zu klein ist.

Fig. 2

EP 0 079 482 A1

**P**ᴀ **ᴇ**ᴛ ᴀɴᴠ ᴀ ᴛ

DIPL.-ING. GERD COMBATZ

7 STUTTGART 1

KLIPPENECKSTRASSE 4 · TELEFON (0711) 465644

21. Oktober 1982 C/F

H 2601 EP

Alois Hoch, Schwärzenbachstr. 7, 7450 Hechingen-Boll

---

## Gerät zum Messen der Elastizität von Langlaufskiern

---

Langlaufskier sollen bekanntlich sowohl Gleiten als auch einen Anstieg ermöglichen. Aus diesem Grunde weisen die Langlaufski in ihrem vorderen und hinteren Bereich eine glatte Lauffläche auf, sind in ihrem mittleren Bereich dagegen mit sogenannten Steighilfen ausgestattet, die entweder durch ein Aufrauhen der Laufflächen oder aber durch ein Belegen dieser Lauffläche mit einem das Gleiten hemmenden Belag zu verwirklichen sind. Damit nun beim Abwärtsgleiten diese Steighilfen der Mittelzone des Langlaufskis möglichst keine Hemmwirkung erzeugen, ist es erforderlich, daß ein solcher Langlaufski in seiner Normalstellung nur mit

-2-

COMMERZBANK STUTTGART NR. 7751027 · POSTSCHECKAMT STUTTGART NR. 25780 · TELEFONISCHE AUSKÜNFTE SIND UNVERBINDLICH

21.10.1982

0079482

seinem vorderen und hinteren Bereich aufliegt, sich
der mittlere Bereich dagegen von der Skispur leicht
abhebt. Dieses Abheben des mittleren Skibereiches
ist aber wieder abhängig sowohl von der Elastizität des
nach oben gewölbten Skis als auch von dem Gewicht
der den betreffenden Ski benutzenden Person. Bei der
Auswahl eines Langlaufskis kommt es also ganz wesentlich darauf an, daß die Länge und damit auch das Abheben
des mittleren Skiteiles von der Unterstützungsfläche
mit dem Gewicht der den Ski benutzenden Person in
Einklang gebracht wird. Im allgemeinen behilft man
sich nun damit, daß die Länge der zu benutzenden Langlaufskier in Abhängigkeit von der Größe der betreffenden
Person ausgewählt werden. Da jedoch bekanntlich gleichgroße Personen oftmals recht unterschiedliche Gewichte
aufweisen, ist eine solche Auswahl doch immer noch
außerordentlich groß und ungenau.

Aufgabe der vorliegenden Erfindung ist es nunmehr, diesen
immer noch bestehenden Übelstand zu beseitigen und
ein Gerät zum Messen der Elastizität von Langlaufskiern zu schaffen, mittels dem genau festgestellt
werden kann, bei welchem Personengewicht nun welcher Skier
geeignet ist.

Diese Aufgabe wird erfindungsgemäß durch ein Gerät gelöst, das durch mindestens eine im Bereich des Skimittelteiles befindliche, auf eine Anzeigeeinrichtung
einwirkende Abstand- oder Abgreifeinrichtung gekennzeichnet ist, beiderseits derselben Auflager für den
vorderen bzw. den hinteren Teil des zu überprüfenden Skis
angeordnet sind. Mittels dieser Abtast- oder Abgreifeinrichtung kann dabei einwandfrei festgestellt werden,
wieweit sich der durch eine Person belastete Skier
gegenüber seiner unbelasteten Stellung absenkt und ob die
damit verbundene Durchbiegung noch als zulässig anzusehen oder aber etwa so groß ist, daß etwa für die betreffende Person ein beispielsweise längerer Ski oder
auch ein härterer und damit weniger elastischer Ski
auszuwählen ist. Dieses Durchbiegen und Absenken des belasteten Skis wird dabei auf die zugeordnete
Anzeigeeinrichtung übertragen, an der sich dann genaue
Werte rasch und einfach ablesen lassen.

Da im allgemeinen nicht ein einzelner Ski, sondern
ein Paar Ski auszuwählen ist, ist es selbstverständlich
zweckmäßig, wenn das Gerät nicht nur mit einer, sondern
mit zwei einander benachbarten Abtast- oder Abgreifeinrichtungen ausgestattet ist, die sich dann gegebenenfalls mittels einer Brücke, einer Lasche od. dgl. miteinander verbinden lassen. Dieser paarweisen Anordnung
derartiger Absperr- oder Abgreifeinrichtung entsprechend kann jeder einzelnen Abtast- oder Abgreifeinrichtung
jeweils eine besondere Anzeigeeinrichtung zugeordnet sein.
Es ist aber auch möglich, daß nur eine gemeinsame Anzeigeeinrichtung vorgesehen ist. Schließlich kann aber neben
den den beiden Abtast- oder Abgreifeinrichtungen
zugeordneten Anzeigeeinrichtungen auch noch eine zusätzliche gemeinsame Anzeigeeinrichtung vorgesehen
sein, die dann wirksam sein wird, wenn beide Abtast-
oder Abgreifeinrichtungen durch die zuvor erwähnte
Brücke miteinander verbunden sind.

Was nun eine Abtast- oder Abgreifeinrichtung betrifft,
so weist diese zweckmäßig eine unter der Wirkung einer
Rückholfeder od. dgl. stehende, vertikal gerichtete
Stößelstange od. dgl. auf, die begrenzt verschiebbar
gelagert ist, an ihrem oberen freien Ende eine an der
Unterfläche des zu messenden Skis anliegende Platte trägt
und mit einem auf die Anzeigeeinrichtung mechanisch,
optisch, pneumatisch oderauch elektrisch einwirkenden
Organ ausgestattet ist.

Ein solches auf die Anzeigeeinrichtung einwirkendes
Organ kann dabei beispielsweise als Zahnstange od. dgl.
ausgebildet sein, die mit dem Ritzel einer die Anzeigeeinrichtung betätigenden Welle kämmt. Desgleichen ist
es aber auch möglich, stattdessen einen über Umlenkrollen

21.10.1982 **0079482**

geführten und unter der Wirkung einer Rückholfeder
stehenden Seilzug od. dgl. vorzusehen, der dann einerseits mit der Stößelstange und andererseits mit der
Anzeigeeinrichtung verbunden ist.

Als Anzeigeeinrichtung selbst kann wieder eine Reihe
elektrischer Lampen vorgesehen sein, die zwar alle an
einer gemeinsamen Stromquelle angeschlossen sind, jedoch
jeweils in einem besonderen Stromkreis mit einem
eigenen Unterbrecherschalter liegen, wobei dann
sämtliche Unterbrecherschalter nacheinander durch das
an der Stößelstange befindliche Organ nacheinander ein-
und ausgeschaltet werden in Abhängigkeit von der jeweiligen
Stellung der verschiebbaren Stößelstange und damit
auch in Abhängigkeitvon der jeweiligen Absenkung des Skimittelteiles.

Bei der Verwendung derartiger Unterbrecherschalter können
beispielsweise die einen Kontakte derselben in einer
Reihe, die zugehörigen Gegenkontakte dagegen in axialer
und radialer Richtung zueinander versetzt an dem Mantel
einer Walze oder Welle angeordnet sein, die der zuvor
erwähnten Kontaktreihe parallel gerichtet ist und von dem
zur Abtast- oder Abgreifeinrichtung gehörigen Organ
in Umlauf versetzt wird.

Als Unterbrecherschalter lassen sich jedoch auch Fotozellen od. dgl. verwenden, die jeweils auf die jeweilige Stellung der verschiebbaren Stößelstange ansprechen.

Handelt es sich um Unterbrecherschalter mit einander gegenüberstehenden Kontakten, so können diese Unterbrecherschalter auch in einer der Stößelstange benachbarten Reihe angeordnet werden, wobei dann die jeweils einander gegenüberstehenden Kontakte wieder jeweils nacheinander durch das zusammen mit der Stößelstange verschiebbare Organ zu schließen sind.

Die Anzeigeeinrichtung kann auch rein mechanisch ausgebildet sein, wobei diese Anzeigeeinrichtung dann mindestens eine entgegen der Wirkung einer Rückholfeder und in Abhängigkeit von dem Hub der Stößelstange drehbare Zeigerwelle aufweist, deren Zeiger eine feststehende, den jeweiligen Meßwert anzeigende Skala zugeordnet ist. In diesem Zusammenhang ist es selbstverständlich auch denkbar, daß an dieser Zeigerwelle dann statt eines Zeigers ein Skalenrad od. dgl. angeordnet und demselben eine feststehende Marke zugeordnet ist.

Bei einer anderen Gestaltung ist es auch noch möglich, daß als Unterbrecherschalter Fotozellen vorgesehen sind, die in einer dem Hub der Stößelstange parallelen Richtung
verlaufenden Reihe angeordnet sind und jeweils auf die
jeweilige Stellung der Stößelstange ansprechen.

Darüber hinaus ist es jedoch auch noch möglich, daß
der Anzeigeeinrichtung eine Vielzahl in einer Reihe
hintereinander liegender und quer zur Reihe gerichteter
optischer oder elektrischer Meßstrecken zugeordnet ist,
die an einem gemeinsamen Meß- oder Anzeigegerät angeschlossen sind und bei dem Hub der Stößelstange nacheinander
von dem an der Stößelstange angeordneten Organ unterbrochen
werden.

Damit auch Skier unterschiedlicher Länge gemessen werden
können, ist es ferner von Vorteil, wenn die oben bereits
erwähnten Auflager in der Längsrichtung des oder der Skier
verschiebbar gelagert und ihre jeweilige Stellung durch
Kennzahlen gekennzeichnet ist.

Um bei solchen Skiern unterschiedlicher Länge jeweils eine
vorbestimmte Lage einzuhalten, ist es auch noch zweckmäßig,
wenn an dem hinteren Auflager jeweils eine Anschlagleiste
für die Hinterkante des zu messenden Skis angeordnet ist
und diese selbst wieder gegenüber dem verschiebbaren
Auflager verschiebbar ist.

Da nun bei Skiern unterschiedlicher Länge auch deren vorderen und hinteren Auflagebereiche unterschiedlich bemessen sind und sich entsprechend der Skilänge vergrößern, ist es zur Erzielung sachlicher Meßergebnisse empfehlenswert, wenn die Verschiebung der zuvor erwähnten Anschlagleiste wieder in einer Abhängigkeit von der Verschiebung des hinteren Auflagers erfolgt. Dieses läßt sich erfindungsgemäß dadurch erreichen, daß sich das hintere Auflager mittels eines lose umlaufenden Ritzels an einer fest an dem Gerät angeordneten Zahnstange abstützt und die durch das Verschieben des Auflagers erzeugte Drehung des Ritzels über eine gegenüberstehende, gegenüber dem Auflager längsverschiebbar gelagerte und mit der Anschlagleiste verbundene Zahnstange auch eine Verschiebung der Anschlagleiste gegenüber des hinteren Auflagers bewirkt wird.

Weitere Einzelheiten der vorliegenden Erfindung ergeben sich aus derfolgenden Beschreibung einer auf der Zeichnung dargestellten beispielsweisen Ausführungsform des Gerätes sowie den sich hieran anschließenden Ansprüchen.

Es zeigen:

Fig. 1   eine Draufsicht des Gerätes,

Fig. 2   eine Seitenansicht desselben,

Fig. 3   einen Schnitt der Tasteinrichtung gemäß der Linie 3-3 in vergrößertem Maßstab.

Fig. 4   einen Schnitt dieser Abtasteinrichtung gemäß der Linie 4-4 und

Fig. 5   einen Längsschnitt gemäß der Linie 5-5 ebenfalls in vergrößertem Maßstab.


Das in der Fig. 1 in der Draufsicht dargestellte Gerät 1 weist einen mit 2 bezeichneten Rahmen auf, auf dem eine Grundplatte 3 aufliegt und der mittels vier Füßen 4 auf der Oberfläche eines mit 5 bezeichneten Bodens aufsitzt. An den beiderseitigen Längsholmen 6 des Rahmens 2 ist zur Unterstützung desselben noch eine aus Profilmaterial gefertigte, im Querschnitt U-förmige Stütze 7 angebracht, die mit ihrem Steg 8 ebenfalls auf dem Boden 5 aufsitzt.


Im vorderen und hinteren Bereich dieser Grundplatte 3 sind beiderseits der Längsholme 6 mit 9 bezeichnete Führungsschienen angebracht, zwischen denen mit 10 und 11 bezeichnete Platten in Richtung der Pfeile 12 längsverschiebbar gelagert und mittels durch in den Führungsschienen 9 befindliche Längsschlitze 13
hindurchgreifender Schrauben 14 od. dgl. gegen ein
Abheben gesichert sind. Diese Platten 10 und 11
dienen dabei als Auflager für die beiden in den
Fig. 1 und 2 nur gestrichelt dargestellten Langlauf-
skier15 und 16. Diese Langlaufskier15 und 16 sind bekannterweise nach oben durchgewölbt, so daß zwischen
deren Mittelteilen 17 und dem Mittelteil 18 der
Grundplatte 3 ein mit 19 bezeichneter Zwischenraum
verbleibt, dessen Höhe in den Fig. 2 bis 4 mit H angegeben ist.

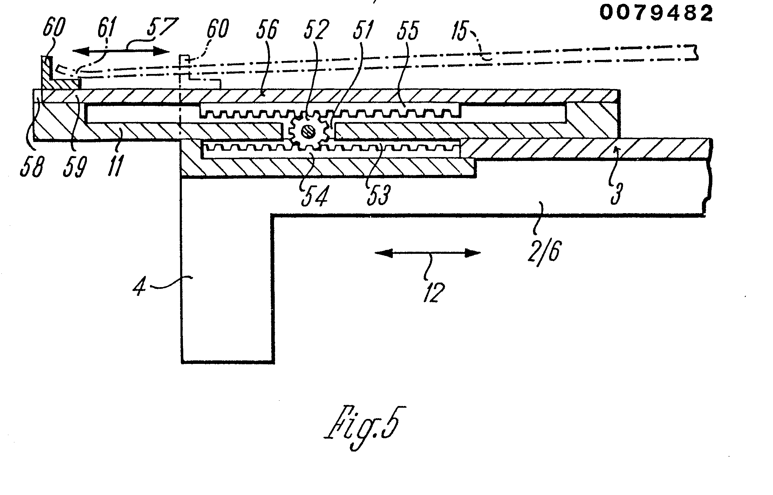

Wie sich insbesondere aus der Fig. 4 ergibt, so
sind in dem Mittelbereich 18 dieses Gerätes 1 zwei
einander benachbarte, mit 20 und 21 bezeichnete
Abtasteinrichtungen angeordnet, von denen die eine
dem Langlaufski 15 und die andere dem benachbarten
Langlaufski 16 zugeordnet ist. Außerhalb dieser beiden
Abtasteinrichtungen 20 und 21 sind auf der Oberfläche
der Grundplatte 3 noch zwei mit 22 bezeichnete Trittplatten zum Aufsetzen der Füße angebracht. Gemäß den
beiden im vergrößerten Maßstab gezeichneten Fig. 3
und 4 weist jede der beiden Abtasteinrichtungen 20
und 21 in dem Bereich der Stütze 7 eine vertikal gerichtete Stößelstange 23 auf, die entgegen der Wirkung
einer Schraubendruckfeder 24 längsverschiebbar in einer an dem Steg 8 der Stütze 7 angebrachten Hülse 25 gelagert ist. Der vertikale Hub dieser Stößelstange 23 ist dabei begrenzt einerseits durch einen sie umgebenden Ringbund 26 und andererseits durch eine an ihrer oberen Stirnseite angebrachten Scheibe 27, wobei der zwischen diesem Ringbund 26 und dieser Scheibe 27 befindliche Schaft 28 dieser Stößelstange 23 durch jeweils eine in dem Mittelteil 18 der Grundplatte 3 befindliche Bohrung 29 hindurchragt. Auf der von der Stößelstange 23 getragenen Scheibe 27 ist eine mit 30 bezeichnete Platte angebracht, auf der ein im Querschnitt U-förmiger Anlageteil 31 sitzt, durch den, wie insbesondere aus der Fig. 4 ersichtlich, der Mittelteil 17 des betreffenden Ski 15,16 hindurchgeführt ist.

Benachbart zu der Scheibe 27 ist an der Unterseite der Platte 30 noch eine nach unten vorstehende und somit der Stößelstange 23 parallel gerichtete Zahnstange 32 angeordnet, die durch eine der Bohrung 29 benachbarte Bohrung 33 hindurchgreift und mit ihrem gezahnten Teil 34 in den unterhalb des Mittelteiles 18 der Grundplatte 3 befindlichen Raum 35 hineinragt. Mit diesem Zahnteil 34 kämmt ein mit 36 bezeichnetes Ritzel, das auf einer in der Fig. 3 dargestellten Welle 37 einer zu einer Anzeigeeinrichtung 38 gehörenden Schalteinrichtung 39 sitzt. Diese Schalteinrichtung 39 weist eine Reihe von sieben feststehenden Kontakten 40 auf,
denen sieben weitere Gegenkontakte 41 zugeordnet sind.
Diese Gegenkontakte 41 sind dabei längs einer Schraubenlinie, also sowohl in radialer Richtung als auch in
axialer Richtung versetzt, auf dem Mantel einer auf
der Welle 37 sitzenden Walze 42 angebracht. Die Kontakte 40
und die zugehörigen Gegenkontakte 41 liegen dabei paarweise jeweils in einem nicht besonders dargestellten
Stromkreis, in dem weiterhin noch jeweils eine von
sieben mit 43 bezeichneten Anzeigelampen liegen, die
ebenfalls zur Anzeigeeinrichtung 38 gehören und jeweils
reihenförmig in der aus den Fig. 1 und 3 ersichtlichen
Weise in der Grundplatte 3 eingelassen sind.

Der Speisung dieser sieben einander parallel geschalteten
Stromkreise dient eine ebenfalls unterhalb der Grundplatte 3 in einem Gehäuse 44 befindliche Stromquelle
in der Form einer Batterie, eines Transformators od. dgl.
Außerdem ist dieser Stromquelle ein mit 45 bezeichneter
Schalter zugeordnet, mittels dem der Stromzufluß zu
den einzelnen Stromkreisen eingeschaltet und unterbrochen werden kann. Benachbart zu diesem Schalter 45
befindet sich noch eine Kontrollampe 46, mittels der
zu erkennen ist, ob diese beispielsweise dem Langlaufski 15 zugeordnete elektrische Anzeigeeinrichtung 38
eingeschaltet ist oder nicht.

Da das erfindungsgemäße Gerät zum Auflegen von zwei einander parallel verlaufenden Langlaufski 15 und 16 ausgelegt ist, ist auch der anderen Abtasteinrichtung 21 eine weitere, gleichartige Anzeigeeinrichtung 47 zugeordnet, die jedoch durch den gleichen Schalter 45 zu bedienen ist.

Ferner ist an der Oberseite der einen Platte 30 noch eine um eine vertikale Achse in Richtung des Pfeiles 48 schwenkbare Brücke 49 angeordnet, mittels der die beiden einander benachbarten Platten 30 und damit auch die beiden Anlageteile 31 miteinander verbunden werden können. Die Verbindung dieser beiden Platten 30 ist dabei immer dann gewünscht, wenn beide Langlaufski 15 und 16 gemeinsam getestet werden sollen. Zu diesem Zweck ist sodann noch eine weitere mit 50 bezeichnete Anzeigeeinrichtung vorgesehen, deren Lampen jeweils in den Stromkreisen der beiderseitig benachbarten Lampen liegen und desgleichen durch den vorerwähnten Schalter 45 einzuschalten sind.

Wie oben schon ausgeführt wurde, so sind die beiden Auflagerplatten 10 und 11 in Richtung der Pfeile 12 längsverschiebbar gelagert, wobei die vordere Auflagerplatte 10 lose gelagert ist, die hintere Auflagerplatte 11 dagegen eine Führung aufweist. Diese Führung besteht in der in der Fig. 5 dargestellten Weise aus einem in einer in der Auflagerplatte 11 befindlichen Aussparung 51 frei drehbar gelagerten Ritzel 52, das in eine in einer in der Grundplatte 3 eingearbeiteten Nut 53 fest eingelegte Zahnstange 54 eingreift. Dieses Ritzel 52 kämmt jedoch zusätzlich noch mit einer oberen Zahnstange 55, die an der Unterseite einer mit 56 bezeichneten Leiste angeordnet ist. Diese Leiste 56 ist wieder längsverschiebbar in Richtung des Pfeiles 57 in einer in der Auflagerplatte 11 befindlichen Längsnut 58 gelagert und trägt an ihrem freien Ende 59 eine mit 60 bezeichnete Anschlagleiste, die in der in den Fig. 2 und 5 dargestellten Weise als Anlage für die Hinterkanten 61 der Langlaufski 15 und 16 dient. Ferner ist in dieser Leiste 56 noch ein Längsschlitz 62 eingearbeitet, durch den der Schaft einer ein unerwünschtes Abheben dieser Leiste 56 verhindernden Schraube 63 hindurchragt.

Die Wirkungsweise dieses Gerätes 1 ist die folgende: Zunächst einmal werden die beiden zu untersuchenden Langlaufski 15 und 16 in der in der Fig. 2 dargestellten Weise auf die beiden Abtasteinrichtungen 20 und 21 aufgelegt, wobei die beiden Mittelteile 17 der beiden Ski 15 und 16 jeweils durch die im Querschnitt U-förmigen Anlageteile 31 hindurchgeführt sind. Jeweils nach der Länge der betreffenden Ski 15 und 16 sind hierbei die Auflagerplatten10 und 11 in die der Skilänge entsprechende Position zu verschieben, wobei auf den Mittelteil 18 der Grundplatte 3 angebrachte Skalen 64 angeben, in welche Stellung diese beiden Auflagerplatten 10 und 11 bei einer bestimmten Skilänge zu bringen sind. Wird nun, wie oben schon angedeutet, die hintere Auflagerplatte 11 in ihrer Längsrichtung verschoben, so hat dieses eine Drehbewegung des Ritzels 52 zur Folge, wodurch der Leiste 56 und damit auch der Anschlagleiste 60 eine zusätzliche Verschiebung in Richtung des Pfeiles 57 erteilt wird. Da der Abstand beider Zahnstangen 54 und 55 von der Achse des Ritzels 52 gleich ist, wird die Leiste 56 dabei um die doppelte Länge des Verschiebeweges der Auflagerplatte 11 verschoben. Diese größere Verschiebung der Anschlagleiste 60 ist dabei erwünscht, um so den jeweils erforderlichen Auflagebereichen bei unterschiedlichen Skilängen gerecht zu werden. Liegen die beiden Langlaufski 15 und 16 in der gewünschten Weise auf den beiden Auflagerplatten 10 und 11 auf, so werden sie mittels um die im Querschnitt U-förmigen Anlageteile 31 herumgreifender Bänder 65 gegen ein unerwünschtes Abheben gesichert.

Nunmehr kann die zur Benutzung der betreffenden Ski 15 und 16 vorgesehene Person sich auf die oben schon erwähnten Trittplatten 22 treten, um von hier aus im
Bereich der Anlageteile 31 auf die Mittelteile 17 der
beiden Ski 15 und 16 umzusteigen. Sobald dieses geschehen
ist, übt die betreffende Person durch ihr Eigengewicht
jeweils einen Druck in Richtung der beiden Pfeile 66
auf die beiden Ski 15 und 16 aus, so daß sich deren
Mittelteile 17 jeweils in der gleichen Richtung absenken.
Mit diesem Absenken werden jedoch auch die Stößelstangen 23
entgegen der Wirkung der Schraubendruckfedern 24
verschoben, was eine entsprechende Verschiebung auch der
beiden Zahnstangen 32 zur Folge hat. Diese beiden
Zahnstangen 32 erteilen dabei den ihnen zugeordneten
Ritzeln 36 eine Drehbewegung in Richtung der Pfeile 67,
was eine Schwenkbewegung der die Gegenkontakte 41
tragenden Walze 42 zur Folge hat. Damit aber kommen
diese Gegenkontakte 41 nacheinander mit den in Reihe
liegenden Kontakten 40 in Berührung, so daß die in
den entsprechenden Stromkreisen liegenden Lampen 43
der zugehörigen Anzeigeeinrichtung 38 bzw. 47 nacheinander
aufleuchten. Sobald die Mittelteile 17 der beiden
Ski 15 und 16 ihre in der Fig. 4 strichpunktiert dargestellten, mit h bezeichneten abgesenkten Stellungen
erreicht haben, leuchten nur noch eine oder zwei
Lampen 43 der betreffenden Anzeigeeinrichtung 38 bzw. 47
auf. Handelt es sich dabei um die Lampen 43 des mittleren Bereiches, so ist der betreffende Ski 15/16 passend. Leuchten dagegen die Lampen 43 am Ende der Anzeigeeinrichtungen 38 bzw. 47 auf, so ist eine zu große Absenkung der beiden Skimittelteile 17 erfolgt. Der betreffende Ski 15/16 ist somit zu elastisch und sollte gegen einen härteren Ski ausgetauscht werden. Leuchten dagegen die vorderen Lampen 43 der Anzeigeeinrichtung auf, so zeigt dieses, daß der Ski 15/16 sich nicht genügend abgesenkt hat und somit für die betreffende Person zu hart ist. In den beiden letzteren Fällen ist somit ein anderer Ski auszuwählen, der dann in der gleichen Weise wie zuvor schon beschrieben von der betreffenden Person zu testen ist.

Eine Variante dieser Untersuchung besteht noch darin, daß beide Platten 30 durch die zuvor schon erwähnte Brücke 49 miteinander verbunden werden können, sodaß nunmehr eine gleichzeitige Überprüfung beider Ski 15 und 16 möglich ist, wobei dann lediglich die mittlere Anzeigeeinrichtung 50 das Meßergebnis wiedergibt.

Bezugszeichenliste

| | | | |
|---|---|---|---|
| 1 | Gerät | 28 | Schaft von 23 |
| 2 | Rahmen | 29 | Bohrungen in 18 |
| 3 | Grundplatte | 30 | Platte |
| 4 | Füße von 2 | 31 | Anlageteil |
| 5 | Boden | 32 | Zahnstange |
| 6 | Längsholme von 2 | 33 | Bohrung zu 32 |
| 7 | Stütze | 34 | gezahnter Teil von 32 |
| 8 | Steg | 35 | Raum |
| 9 | Führungsschiene von 10/11 | 36 | Ritzel |
| 10 | Auflagerplatte vorn | 37 | Welle |
| 11 | Auflagerplatte hinten | 38 | Anzeigeeinrichtung |
| 12 | Pfeil | 39 | Schalteinrichtung |
| 13 | Längsschlitze in9 | 40 | Kontakte |
| 14 | Schrauben | 41 | Gegenkontakte |
| 15 | Langlaufski | 42 | Walze |
| 16 | Langlaufski | 43 | Anzeigelampen |
| 17 | Mittelteil von 15/16 | 44 | Gehäuse |
| 18 | Mittelteil von 3 | 45 | Schalter |
| 19 | Zwischenraum | 46 | Kontrollampen |
| 20 | Abtasteinrichtung zu 15 | 47 | Anzeigeeinrichtung |
| 21 | Abtasteinrichtung zu 16 | 48 | Pfeil |
| 22 | Trittplatten | 49 | Brücke |
| 23 | Stößelstangen von 20/21 | 50 | Anzeigeeinrichtung |
| 24 | Schraubendruckfeder | 51 | Aussparung in 11 |
| 25 | Hülse zu 23 | 52 | Ritzel |
| 26 | Ringbund von 23 | 53 | Nut |
| 27 | Scheibe von 23 | | |

Bezugszeichenliste

54 Zahnstange untere

55 Zahnstange obere

56 Leiste zu 55

57 Pfeil

58 Längsnut zu 56

59 Ende von 56

60 Anschlagleiste

61 Hinterkanten von 15/16

62 Längsschlitz in 56

63 Schraube zu 56

64 Skalen

65 Bänder zu 31

66 Pfeil

67 Pfeil


H = Höhe von 19

h = abgesenkte Höhe von 17

7 STUTTGART I
KLIPPENECKSTRASSE 4 · TELEFON (0711) 465644

23. Oktober 1981 C/F

H 2601 EP

Alois Hoch, Schwärzenbachstr. 7, 7450 Hechingen-Boll

Gerät zum Messen der Elastizität von Langlaufskiern

Ansprüche:

1. Gerät zum Messender Elastizität von Langlaufskieren,
   gekennzeichnet durch mindestens eine im Bereich des Skimittelteiles (17) befindliche, auf eine Anzeigeeinrichtung (38,47,50) einwirkende Abtast- oder Abgreifeinrichtung (20,21), beiderseits derselben Auflager (10,11)
   für den vorderen bzw.den hinteren Teil des zu messenden
   Ski (15,16) angeordnet sind.

2. Gerät nach Anspruch 1, dadurch gekennzeichnet, daß
   dieses (1) mit zwei einander benachbarten Abtast- oder
   Abgreifeinrichtungen (20,21) ausgerüstet ist.

-2*-

·OMMERZBANK STUTTGART NR. 7751027 · POSTSCHECKAMT STUTTGART NR. 25780 · TELEFONISCHE AUSKÜNFTE SIND UNVERBINDLICH

**0079482**

3. Gerät nach Anspruch 2, dadurch gekennzeichnet, daß die einander benachbarten Abtast- oder Abgreifeinrichtungen (20,21) mittels einer Brücke (49), einer Lasche od. dgl. miteinander zu verbinden sind.

4. Gerät nach Anspruch 2 und/oder 3, dadurch gekennzeichnet, daß den einander benachbarten Abtast- oder Abgreifeinrichtungen (20,21) jeweils eine besondere Anzeigeeinrichtung (38,47) zugeordnet ist.

5. Gerät nach Anspruch 3, dadurch gekennzeichnet, daß den einander benachbarten Abtast- und Abgreifeinrichtungen (20,21) nur eine gemeinsame Anzeigeeinrichtung (50) zugeordnet ist.

6. Gerät nach Anspruch 2 und/oder 3, dadurch gekennzeichnet, daß den einander benachbarten Abtast- oder Abgreifeinrichtungen (20,21) jeweils eine eigene Anzeigeeinrichtung (38,47), darüber hinaus aber auch noch eine gemeinsame Anzeigeeinrichtung (50) vorgesehen ist.

7. Gerät nach einem oder mehreren der Ansprüche 1 bis 6, dadurch gekennzeichnet, daß jede der Abtast- oder Abgreifeinrichtungen (20,21) eine unter der Wirkung einer Rückholfeder (24) od. dgl. stehende, vertikal gerichtete Stößelstange (23) od. dgl. aufweist, die begrenzt verschiebbar gelagert ist, an ihrem oberen freien Ende eine Anlageplatte (30,31) trägt und mit einem auf die Anzeigeeinrichtung (38,47,50) mechanisch, optisch, pneumatisch oder elektrisch einwirkenden Organ (32) ausgestattet ist.


8. Gerät nach Anspruch 7, dadurch gekennzeichnet, daß als auf die Anzeigeeinrichtung (38,47,50) einwirkendes Organ eine Zahnstange (32) od. dgl. vorgesehen ist, die mit dem Ritzel (36) einer die Anzeigeeinrichtung (38,47,50) betätigenden Welle (37) kämmt.


9. Gerät nach Anspruch 7, dadurch gekennzeichnet, daß als auf die Anzeigeeinrichtung (38,47,50) einwirkendes Organ ein über Umlenkrollen geführter und unter der Wirkung einer Rückholfeder stehender Seilzug od. dgl.

vorgesehen ist, der einerseits mit der Stößelstange (23) und andererseits mit der Anzeigeeinrichtung (38,47,50) verbunden ist.


10. Gerät nach einem oder mehreren der Ansprüche 7 bis 9, dadurch gekennzeichnet, daß als Anzeigeeinrichtung (38,47,50) eine Reihe elektrischer Lampen (43) vorgesehen ist, die zwar alle an einer gemeinsamen Stromquelle (44) angeschlossen sind, jedoch jeweils in einem besonderen Stromkreis mit einem eigenen Unterbrecherschalter (40/41) liegen, wobei dann sämtliche Unterbrecherschalter (40,41) nacheinander durch das an der Stößelstange (23) befindliche Organ (32) ein- und ausschaltbar sind in Abhängigkeit von der jeweiligen Stellung der verschiebbaren Stößelstange (23).

11. Gerät nach Anspruch 10, dadurch gekennzeichnet, daß die einen Kontakte (40) der Unterbrecherschalter in einer Reihe und die zugehörigen Gegenkontakte (41) in axialer und radialer Richtung zueinander versetzt an dem Mantel einer Walze (42) oder Welle (37) angeordnet sind, die der Kontaktreihe (40) parallel gerichtet ist und von dem zur Abtast- und Abgreifeinrichtung (20,21) gehörigen Organ (32) in Umlauf (67) versetzt wird.

12. Gerät nach Anspruch 10, dadurch gekennzeichnet, daß die Unterbrecherschalter in einer der Stößelstangen (23) benachbarten Reihe angeordnet sind und ihre jeweils einander gegenüberstehenden Kontakte jeweils nacheinander das zusammen mit der Stößelstange (23) verschiebbare Organ (32) zu schließen sind.

13. Gerät nach einem oder mehreren der Ansprüche 7 bis 9, dadurch gekennzeichnet, daß die Anzeigeeinrichtung (20,21) mindestens eine entgegen der Wirkung einer Rückholfeder und in Abhängigkeit von dem Hub der Stößelstange (23) drehbare Zeigerwelle aufweist, deren Zeiger eine feststehende, den jeweiligen Meßwert anzeigende Skala zugeordnet ist.

14. Gerät nach Anspruch 13, dadurch gekennzeichnet, daß an der Zeigerwelle statt eines Zeigers ein Skalenrad od. dgl. angeordnet und dem-selben eine feststehende Marke zugeordnet ist.

15. Gerät nach Anspruch 10, dadurch gekennzeichnet, daß als Unterbrecherschalter Fotozellen vorgesehen sind, die in einer der Längsrichtung der Stößelstange (23) parallelen Richtung verlaufenden Reihe angeordnet sind und jeweils auf die jeweilige Stellung der Stößelstange (23) ansprechen.

16. Gerät nach einem oder mehreren der Ansprüche 7 bis 9, dadurch gekennzeichnet, daß der Anzeigeeinrichtung (38,47,50) eine Vielzahl in einer Reihe hintereinander liegender und quer zu dieser Reihe gerichteter optischer oder elektrischer Meßstellen zugeordnet ist, die an einem gemeinsamen Meß- und/oder Anzeigegerät angeschlossen sind und bei dem Hub der Stößelstange (23) nacheinander von dem an der Stößelstange (23) angeordneten Organ unterbrochen werden.

17. Gerät nach Anspruch 1, dadurch gekennzeichnet, daß die
Auflager (10,11) in der Längsrichtung des oder der
Skier (15,16) verschiebbar (12) gelagert und ihre
jeweilige Stellung durch Kennzahlen (64) gekennzeichnet
ist.

18. Gerät nach Anspruch 17, dadurch gekennzeichnet, daß
an dem hinteren Auflager (11) eine Anschlagleiste (60)
für die Hinterkante (61) des zu messenden Skis (15/16)
angeordnet ist.

19. Gerät nach Anspruch 18, dadurch gekennzeichnet, daß die
Anschlagleiste (60) selbst auch noch gegenüber dem
verschiebbaren Auflager (11) verschiebbar (57) ist.

20. Gerät nach Anspruch 19, dadurch gekennzeichnet, daß sich
das hintere Auflager (11) mittels eines lose umlaufenden
Ritzels (52) an einer fest an dem Gerät (1) angeordneten Zahnstange (54) abstützt und die durch das Verschieben des Auflagers (11) erzeugte Drehung dieses
Ritzels (52) über eine gegenüberstehende, gegenüber dem
Auflager (11) längsverschiebbar gelagerte und mit
der Anschlagleiste (60) verbundene Zahnstange (55)
eine Verschiebung der Anschlagleiste (60) gegenüber
dem Auflager (11) bewirkt.

Fig.1

Fig.2

0079482

*Fig.3*

*Fig.4*

0079482

*Fig.5*

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | --- | | G 01 M  5/00 |
| X | WO-A-7 900 207  (S. NORBERG) | 1,2,10 ,18 | |
| | * Ansprüche 1-6 * | | |
| | . | | |
| | --- | | |
| X | US-A-4 290 499  (M. LUOMARANTA) | 1 | |
| | * Ansprüche 1-6 * | | |
| | --- | | |
| A | DE-A-2 535 775  (E. PLENK GMBH & CO.) | | |
| | * Ansprüche 1, 2, 6, 7; Figuren 1, 2 * | | |
| | ----- | | |

|  | RECHERCHIERTE SACHGEBIETE (Int. Cl. 3) |
|---|---|
| | G 01 L   5/00 |
| | G 01 M   5/00 |

Der vorliegende Recherchenbericht wurde für alle Patentansprüche erstellt.

| Recherchenort | Abschlußdatum der Recherche | Prüfer |
|---|---|---|
| BERLIN | 13-01-1983 | KOEHN G |